# EUROPÄISCHE PATENTANMELDUNG

(11) **EP 3 560 417 A1**
(43) Veröffentlichungstag der Anmeldung: **30.10.2019**
(21) Anmeldenummer: 18169768.1
(22) Anmeldetag: 27.04.2018
(51) Int. Cl.: A61B 5/01, A61B 18/14, A61B 18/00

(54) **ÖSOPHAGUSSONDE UND SYSTEM**

(71) Anmelder: VascoMed GmbH, 79589 Binzen (DE)
(72) Erfinder: Ebert, Henning, 12437 Berlin (DE); Bartels, Marc, 10115 Berlin (DE)
(74) Vertreter: Engelhardt, Björn

(57) **Zusammenfassung**

Es ist eine Ösophagussonde (8) zum Bestimmen der Temperatur und/oder der Temperaturverteilung im Ösophagus (26) offenbart. Die Sonde umfasst einen Körper (20), einen zumindest abschnittsweise in dem Körper (20) angeordneten Lichtwellenleiter (21) und einen in dem Lichtwellenleiter (21) gebildeten Interferenzfilter (22). Weiterhin ist ein System mit einer Ösophagussonde (8), einer Lichtquelle (6) und einer Auswerteeinheit (7) offenbart.

## Beschreibung

Die Offenbarung betrifft eine Ösophagussonde zum Bestimmen der Temperatur und/oder Temperaturverteilung im Ösophagus sowie ein System.

### Hintergrund

In der Elektrophysiologie werden bei Arrhythmien beispielsweise mit Hilfe von Hochfrequenz (radio frequency, RF) - Ablationen ungewünschte/pathologische elektrische Verbindungen im Herzen verödet. Hierzu wird die Elektrode eines RF-Ablationskatheters innerhalb des Herzens an der gewünschten Stelle positioniert und Gewebe im Herzen mit RF-Energie abladiert. Eine zweite Elektrode (Neutralelektrode) ist großflächiger und befindet sich in der Regel auf dem Rücken des Patienten.

Insbesondere bei Ablationen im linken Vorhof besteht das Risiko, die Speiseröhre thermisch zu verletzten und eine Fistel (Atrial-Esophageal Fistula) zu erzeugen. Um dies zu verhindern, werden Ösophagussonden verwendet, die während der RF-Ablation die Temperatur in der Speiseröhre messen.

Bei der Verwendung metallischer Temperatursensoren entsteht jedoch eine Antennenwirkung, wodurch die Sensorelemente selbst eine Wärmequelle darstellen. Des Weiteren können eine unsachgemäße Bedienung bei Verwendung des gleichen Erdpotentials sowie eine Läsionsausbreitung durch höhere Wärmeleitfähigkeit von Metall problematisch sein.

Die Dokumente US 2010/0030098 A1 und US 8,145,289 B2 offenbaren die Nutzung einer Vielzahl hintereinander liegender Temperatursensoren zum Bestimmen der Temperatur.

Das Dokument US 2013/0006139 A1 offenbart die Nutzung zweier Temperatursensoren und einer Elektrode zur Generierung einer ständig aktualisierten dreidimensionalen (3D) anatomischen Karte der Speiseröhre mit Temperaturinformationen zur Einbindung in ein 3D-Mapping System.

Die Dokumente US 2014/0012155 A1, WO 2015/187626 A1 und US 2015/0342463 A1 offenbaren Vorrichtungen zum Bestimmen der Temperatur im Ösophagus mittels Infrarotstrahlung. Ein optischer Sensor bestimmt anhand der detektierten Infrarotstrahlung die Temperatur.

### Zusammenfassung

Aufgabe ist es, verbesserte Technologien zum Bestimmen der Temperatur im Ösophagus anzugeben. Insbesondere soll eine Erwärmung der Messvorrichtung vermieden werden.

Es sind eine Ösophagussonde nach Anspruch 1 und ein System nach Anspruch 15 offenbart. Weitere Ausführungsformen sind Gegenstand von abhängigen Ansprüchen.

Nach einem Aspekt ist eine Ösophagussonde zum Bestimmen der Temperatur und/oder Temperaturverteilung im Ösophagus bereitgestellt. Die Ösophagussonde weist einen (z. B. langgestreckten) Körper, einen zumindest abschnittsweise in dem Körper angeordneten Lichtwellenleiter und einen in dem Lichtwellenleiter gebildeten Interferenzfilter auf.

Nach einem weiteren Aspekt ist ein System offenbart. Das System umfasst eine Ösophagussonde, eine Lichtquelle und eine Auswerteeinheit. Die Lichtquelle ist mit einem proximalen Ende des Lichtwellenleiters gekoppelt und ist eingerichtet, Licht in den Lichtwellenleiter einzubringen. Die Auswerteeinheit ist mit dem Lichtwellenleiter gekoppelt und ist eingerichtet, auf Grundlage des von dem Lichtwellenleiter abgegebenen Lichts eine Temperatur zu bestimmen.

Die Ösopagussonde kann frei von metallischen Komponenten sein.

Lichtwellenleiter sind aus Lichtleitern bestehende Leitungen zur Übertragung von Licht. Das Licht wird dabei in Fasern aus beispielsweise Quarzglas oder Kunststoff (polymere optische Faser) geführt. Lichtwellenleiter sind dielektrische Wellenleiter. Sie können aus konzentrischen Schichten aufgebaut sein, wobei im Zentrum ein lichtführender Kern angeordnet ist, der von einem Mantel umgeben ist. Der Mantel hat einen niedrigeren Brechungsindex als der Kern.

Die Lichtquelle kann eingerichtet sein, Licht mit verschiedenen Wellenlängen abzugeben, wobei die Wellenlänge des abgegebenen Lichts auswählbar ist. Die Lichtquelle kann ein Laser sein.

Die Auswerteeinheit kann einen Prozessor und einen Speicher umfassen. Der Prozessor kann eingerichtet sein, anhand der Wellenlänge des von dem Lichtwellenleiter empfangenen Lichts eine Temperatur zu bestimmen.

Die Auswerteeinheit kann mit einer Anzeigeeinrichtung gekoppelt sein. Die Auswerteeinheit kann eingerichtet sein, den ermittelten Wert der Temperatur auf der Anzeigeeinrichtung darzustellen.

Der Interferenzfilter kann ein Faser-Bragg-Gitter (FBG) sein. Faser-Bragg-Gitter sind in Lichtwellenleiter eingeschriebene optische Interferenzfilter. Sie können mit einem Laser (z. B. UV Femtosekunden-Laser) in den Kern des Lichtwellenleiters eingeschrieben werden. Wellenlängen, die innerhalb der Filterbandbreite des FBG liegen, werden reflektiert. Bei einer Änderung der Temperatur ändert sich der Gitterabstand des FBG. Diese Änderung führt zu einer Änderung der Wellenlänge des reflektierten Lichts. Anhand der gemessenen Wellenlänge kann die Temperatur bestimmt werden.

In dem Lichtwellenleiter können mehrere voneinander beabstandete Interferenzfilter gebildet sein. Beispielsweise können mehrere Faser-Bragg-Gitter in den Lichtwellenleiter eingeschrieben sein. Bei jeder in der Anmeldung offenbarten Ausführungsform kann der Interferenzfilter (oder ggf. mehrere Interferenzfilter) als Faser-Bragg-Gitter ausgeführt sein. Die Verwendung von mehreren Interferenzfiltern ermöglicht eine Auswertung mehrerer Messorte im Ösophagus. Hierdurch kann beispielsweise ein Verrutschen der Ösophagussonde ausgeglichen werden.

In dem Körper der Ösophagussonde können mehrere Lichtwellenleiter angeordnet sein (z. B. nebeneinander oder als Bündel), wobei in jedem der mehreren Lichtwellenleiter wenigstens ein Interferenzfilter gebildet sein kann. In jedem der mehreren Lichtwellenleiter können mehrere Interferenzfilter gebildet sein. Die mehreren Lichtwellenleiter können eine gleiche Anzahl von Interferenzfiltern aufweisen. Es kann auch vorgesehen sein, dass die mehreren Lichtwellenleiter unterschiedlich viele Interferenzfilter aufweisen.

Der Lichtwellenleiter oder die mehreren Lichtwellenleiter können vollständig in dem Körper angeordnet sein. Es kann ebenfalls vorgesehen sein, dass ein Abschnitt des Lichtwellenleiters oder der mehreren Lichtwellenleiter aus dem Körper herausragt, beispielsweise an einer distalen Öffnung des Körpers. Interferenzfilter können in einem Abschnitt des Lichtwellenleiters angeordnet sein, der sich im Körper befindet. Alternativ oder ergänzend können Interferenzfilter in dem aus dem Körper herausragenden Abschnitt des Lichtwellenleiters/der Lichtwellenleiter gebildet sein.

Die mehreren Interferenzfilter können unterschiedliche Filterbandbreiten aufweisen, sowohl innerhalb eines Lichtwellenleiters als auch ggf. in verschiedenen Lichtwellenleitern. Bei Verwendung von Faser-Bragg-Gittern können diese unterschiedliche Gitterabstände haben.

Der Lichtwellenleiter kann zumindest abschnittsweise oder vollständig von einem Mantel umgeben sein. Der Mantel kann Schutz vor mechanischen Belastungen gewährleisten. Der (oder ggf. die mehreren) Interferenzfilter kann (können) an der Oberfläche des Mantels angeordnet sein, um eine Wärmeübertragung zu gewährleisten.

Der Interferenzfilter kann von einem Versteifungsmittel umgeben sein. Das Versteifungsmittel kann verhindern, dass Effekte aufgrund einer mechanischen Belastung (z. B. Dehnung oder Stauchung) zu einer Veränderung der Wellenlänge des Lichts führen. Wenn mehrere Interferenzfilter vorgesehen sind, kann jeder Interferenzfilter von einem zugeordneten Versteifungsmittel umgeben sein.

Die Ösophagussonde kann ferner ein Markierungselement aufweisen, das eine Position des Interferenzfilters angibt. Das Markierungselement kann beispielsweise als Röntgenmarker oder als Magnetsensor ausgeführt sein. Wenn mehrere Interferenzfilter vorgesehen sind, kann jedem Interferenzfilter ein Markierungselement zugeordnet sein, das die jeweilige Position des zugeordneten Interferenzfilters angibt.

Es kann vorgesehen sein, dass der Körper der Ösophagussonde derart gestaltet ist, dass ein Kontakt der Interferenzfilter mit einer Innenwand des Ösophagus gewährleistet ist. Einige beispielhafte Ausführungsformen hierfür werden im Folgenden erläutert.

Der Körper mit dem Lichtwellenleiter kann eine Krümmung aufweisen. Der Körper kann mehrfach gekrümmt sein. Die Krümmungen können mit abwechselnden Richtungen (z. B. schlangenförmig) ausgeführt sein. Es kann vorgesehen sein, dass in jedem gekrümmten Abschnitt des Körpers (und damit des Lichtwellenleiters) jeweils ein oder mehrere Interferenzfilter angeordnet sind.

Der Körper mit dem Lichtwellenleiter kann spiralförmig sein.

Der Lichtwellenleiter kann an einem distalen Ende des Körpers eine Schleife bilden.

In dem Körper können mehrere Lichtwellenleiter angeordnet sein, wobei die mehreren Lichtwellenleiter an einem distalen Ende des Körpers eine Korbstruktur bilden, und wobei im Bereich der Korbstruktur in jedem der mehreren Lichtwellenleiter wenigstens ein Interferenzfilter gebildet ist. In jedem Lichtwellenleiter können auch mehrere Interferenzfilter gebildet sein.

In dem Körper können mehrere Lichtwellenleiter angeordnet sein, wobei die mehreren Lichtwellenleiter an einem distalen Ende des Körpers auf einem Ballon angeordnet sind und wobei im Bereich des Ballons in jedem der mehreren Lichtwellenleiter wenigstens ein Interferenzfilter gebildet ist. In jedem Lichtwellenleiter können mehrere Interferenzfilter gebildet sein.

In dem Körper können mehrere Lichtwellenleiter derart angeordnet sein, dass die mehreren Lichtwellenleiter einen Innenraum umgeben, wobei in jedem der mehreren Lichtwellenleiter wenigstens ein Interferenzfilter gebildet ist. In jedem Lichtwellenleiter können auch mehrere Interferenzfilter gebildet sein.

### Beschreibung von Ausführungsbeispielen

Im Folgenden werden beispielhafte Ausführungsformen unter Bezugnahme auf Figuren näher erläutert. Es zeigen:
- Fig. 1: eine schematische Prinzipskizze des Aufbaus einer FBG-Faser,
- Fig. 2: einen schematischen Aufbau einer Ösophagussonde,
- Fig. 3: einen schematischen Aufbau eines Systems zum Bestimmen der Temperatur im Ösophagus,
- Fig. 4: eine gekrümmte Ausführungsform der Ösophagussonde,
- Fig. 5: eine spiralförmige Ausführungsform der Ösophagussonde,
- Fig. 6: eine Ausführungsform der Ösophagussonde mit einem schleifenförmigen Ende,
- Fig. 7: eine korbartige Ausführungsform der Ösophagussonde,
- Fig. 8: eine Ausführungsform der Ösophagussonde mit einem Ballon und
- Fig. 9: eine röhrenförmige Ausführungsform der Ösophagussonde.

Im Folgenden werden für gleiche Komponenten die gleichen Bezugszeichen verwendet.

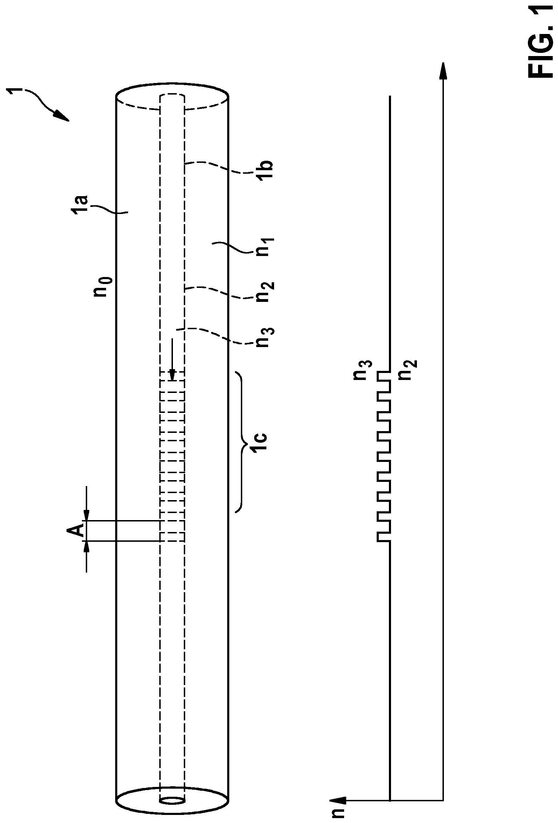

Fig. 1 zeigt im oberen Teil als schematische perspektivische Darstellung einen Abschnitt einer FBG-Faser 1 (Lichtwellenleiter), die eingebettet in einen Mantel 1a mit einem Brechungsindex n1, einen Kern 1b hat, dessen Brechungsindex im Wesentlichen über die gesamte Längserstreckung n2 ist. In einem Sensorabschnitt 1c der Faser 1 ist ein Bragg-Gitter mit einem Gitterabstand A ausgebildet, in dem Abschnitte mit dem Brechungsindex n2 sich mit Abschnitten eines anderen Brechungsindex n3 abwechseln, wie im unteren Teil der Figur 1 verdeutlicht. Der Sensorabschnitt 1c der Faser 1 kann unter anderem zur Erfassung von Temperaturänderungen genutzt werden, wie aus dem Stand der Technik bekannt ist und daher hier nicht näher beschrieben wird. Faser-Bragg-Gitter sind bekannt und werden unter anderem in Ablationskathetern zur Bestimmung der Kraft und der Temperatur verwendet (siehe beispielsweise EP 2 711 676 A2).

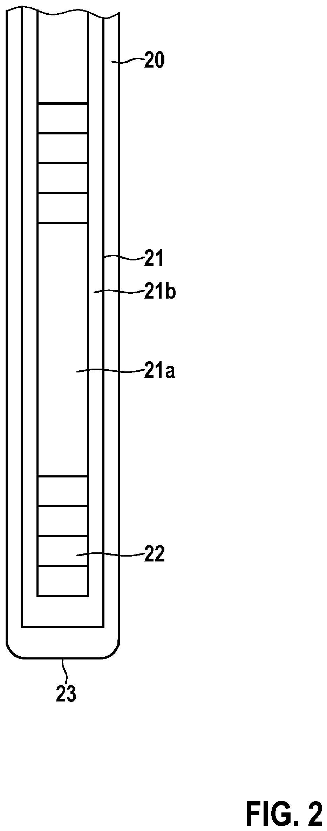

Fig. 2 zeigt eine schematische Darstellung einer Ösophagussonde mit einem Körper 20. In dem Körper 20 ist ein Lichtwellenleiter 21 angeordnet, der einen Kern 21a und einen Mantel 21b aufweist. In dem Lichtwellenleiter sind Faser-Bragg-Gitter 22 gebildet. Bei der gezeigten Ausführungsform sind zwei FBGs 22 gezeigt, es kann jedoch jede beliebige Anzahl von FBGs vorgesehen sein. Ein distales Ende 23 des Körpers 20 ist mit abgerundeten Ecken gebildet, um ein Verletzungsrisiko bei der Anwendung der Sonde zu verringern. Der Durchmesser der Ösophagussonde kann kleiner als 9 Fr (French) sein, bevorzugt kleiner als 7 Fr. Die Länge der Ösophagussonde kann mehr als 20 cm betragen.

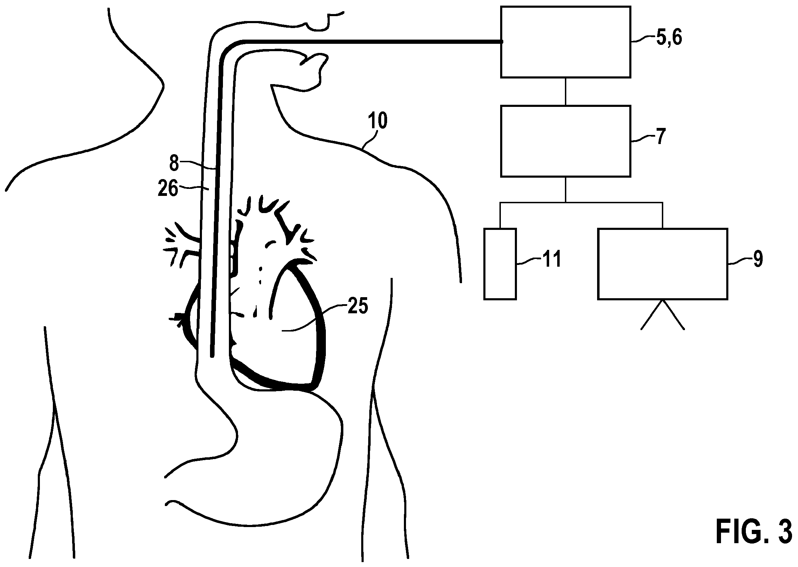

Eine schematische Abbildung eines Systems zum Bestimmen der Temperatur im Ösophagus ist in Fig. 3 dargestellt. Eine Ösophagussonde 8 ist in den Ösophagus 26 eines Patienten 10 eingeführt, um während einer Ablation im Herzen 25 die Temperatur im Ösophagus 26 zu bestimmen. Die Ösophaugssonde 8 ist mit einer Lichtquelle 5 (z. B. einem Laser) gekoppelt. Mit der Lichtquelle 5 wird Licht in den (oder die) Lichtwellenleiter der Ösophagussonde 8 eingekoppelt. Die Wellenlängen des eingekoppelten Lichts können beispielsweise im Bereich von 600 nm - 1650 nm liegen, etwa, aber nicht ausschließlich, bei 1550 ± 50 nm oder 850 ± 50 nm.

Reflektiertes Licht, das aus dem Lichtwellenleiter austritt, wird von einem Detektor 6 (z. B. Spektrometer) aufgefangen und analysiert. In der gezeigten Ausführungsform sind die Lichtquelle 5 und der Detektor 6 in einem Gerät integriert. Die beiden Komponenten können auch separat ausgebildet sein. Der Detektor 6 ist mit einer Auswerteeinheit 7 (z. B. einem Computer) gekoppelt. Die Auswerteeinheit 7 ist eingerichtet, anhand des vom Detektor 6 empfangenen reflektierten Lichts eine Temperatur zu bestimmen. Die Auswerteeinheit 7 kann auch mit der Lichtquelle 5 gekoppelt sein und beispielsweise die Lichtabgabe (z. B. Wellenlänge und/oder Pulsdauer) zu steuern. Die Auswerteeinheit 7 ist mit einer Anzeigeeinrichtung 9 (z. B. einem Bildschirm) gekoppelt. Die Auswerteeinheit 7 ist eingerichtet, den Wert der Temperatur auf der Anzeigeeinrichtung 9 darzustellen.

Optional ist ein Anschluss für ein Thermoelement 11 vorgesehen, der mit der Auswerteeinheit 7 gekoppelt ist. Hiermit ist es möglich, die Maximaltemperatur der Ösophagussonde an ein weiteres Gerät als Thermospannung zu übergeben. Damit können handelsübliche Geräte (z. B. RF-Generatoren) die Temperaturinformation nutzen.

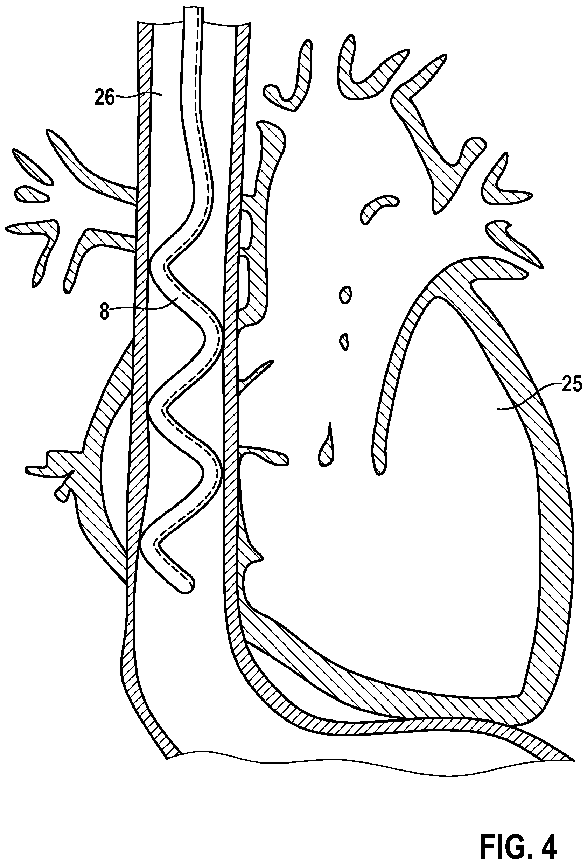

Fig. 4 zeigt eine gekrümmte Ausführungsform der Ösophagussonde 8. Aus Gründen der Übersichtlichkeit sind hier die einzelnen Komponenten (Körper, Lichtwellenleiter und Faser-Bragg-Gitter) nicht dargestellt, obwohl diese selbstverständlich vorhanden sind. Die Ösophagussonde 8 weist mehrere gegenläufige Krümmungen auf, so dass ein schlangenförmiger Verlauf der Sonde gebildet ist. Hierdurch kann die Ösophagussonde in mehreren außen liegenden Bereichen mit der Innenwand des Ösophagus 8 in Kontakt kommen.

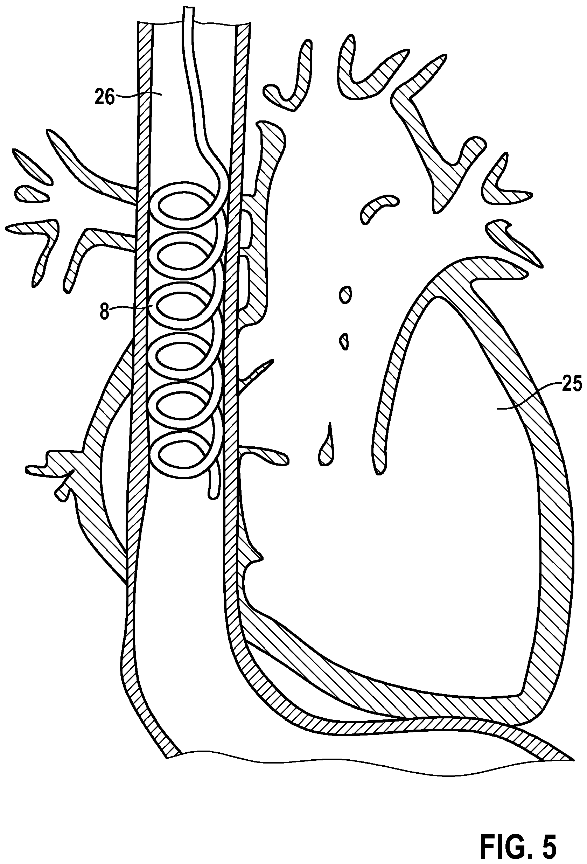

In Fig. 5 ist eine Ösophagussonde 8 mit einem spiralförmigen Körper gezeigt. Auch hier sind zur besseren Übersichtlichkeit die einzelnen Komponenten (Körper, Lichtwellenleiter und Faser-Bragg-Gitter) nicht dargestellt.

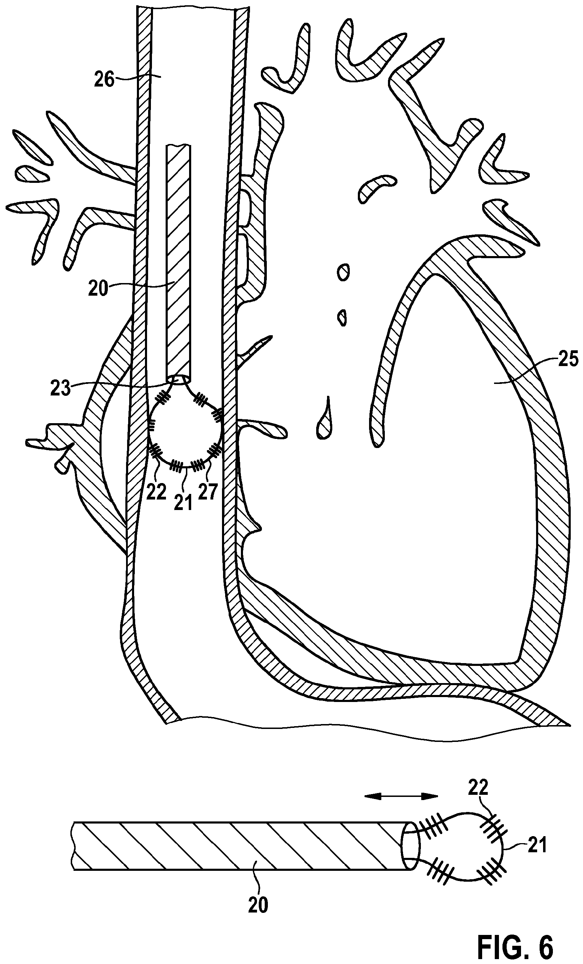

Fig. 6 zeigt im oberen Teil eine Ösophagussonde mit einer Schleife 27, welche an einem distalen Ende 23 des Körpers 20 herausragt. An der Schleife 27 sind mehrere FBGs 22 gebildet. Im unteren Teil von Fig. 6 ist gezeigt, wie die Schleife 27 in den Körper 20 eingezogen/herausgeschoben wird.

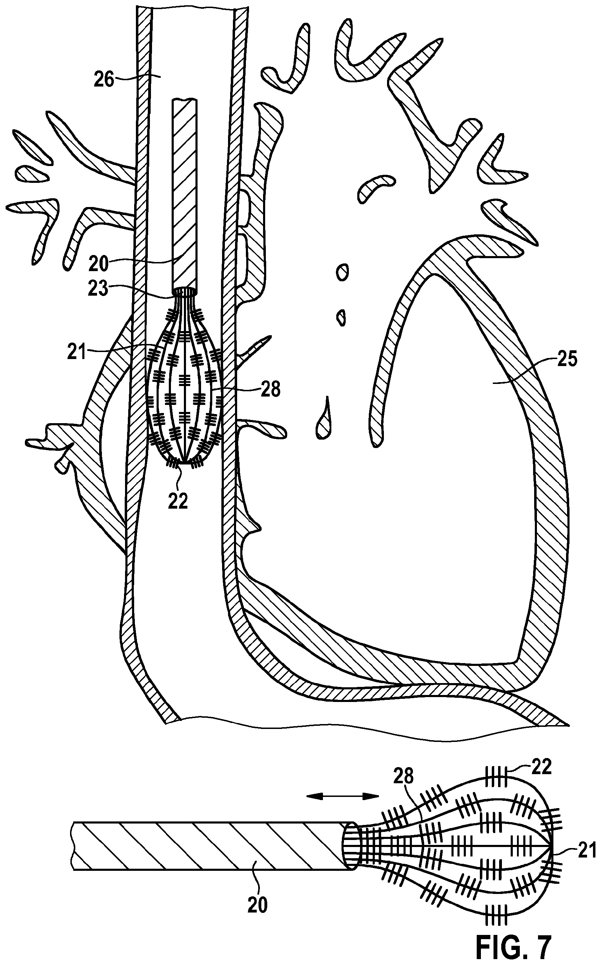

Im oberen Teil von Fig. 7 ist eine Ösophagussonde mit einer korbartigen Struktur 28 gezeigt. Mehrere Lichtwellenleiter 21 treten aus einer Öffnung am distalen Ende 23 des Körpers 20 aus. Die Lichtwellenleiter sind an einem Punkt miteinander verbunden und spannen einen Korb 28 ("basket") auf. Der untere Teil von Fig. 7 stellt das Einziehen/Ausfahren des Korbs 28 in den Körper 20 dar.

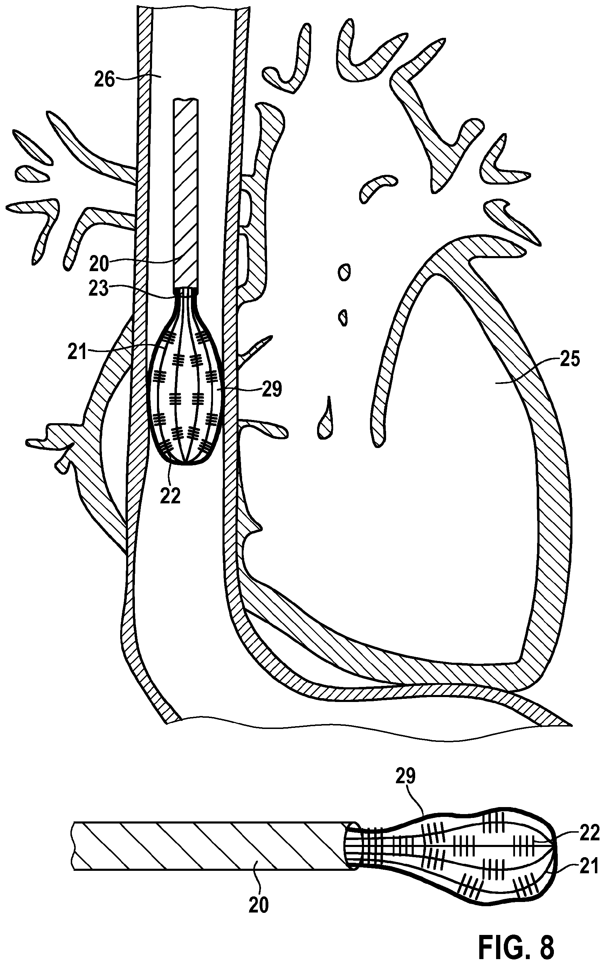

Fig. 8 zeigt im oberen Teil eine Ösophagussonde mit einem Ballon 29, der aus einem distalen Ende 23 des Körpers 20 herausragt. Auf dem Ballon 29 sind mehrere Lichtwellenleiter 21 mit FBGs 22 angeordnet. Im unteren Teil von Fig. 8 ist der Ballon 29 im erschlafften (nicht gefüllten) Zustand beim Einziehen/Ausfahren dargestellt. Der Ballon 29 kann beispielsweise mit einer Salzlösung, z.B. mit einer isotonischen Kochsalzlösung wie Natriumchlorid-Infusionslösung (0.9%), gefüllt werden, so dass er sich ausdehnt. Diese Salzlösung kann über eine Zuleitung im Körper 20 zugeführt und/oder abgepumpt werden (nicht dargestellt).

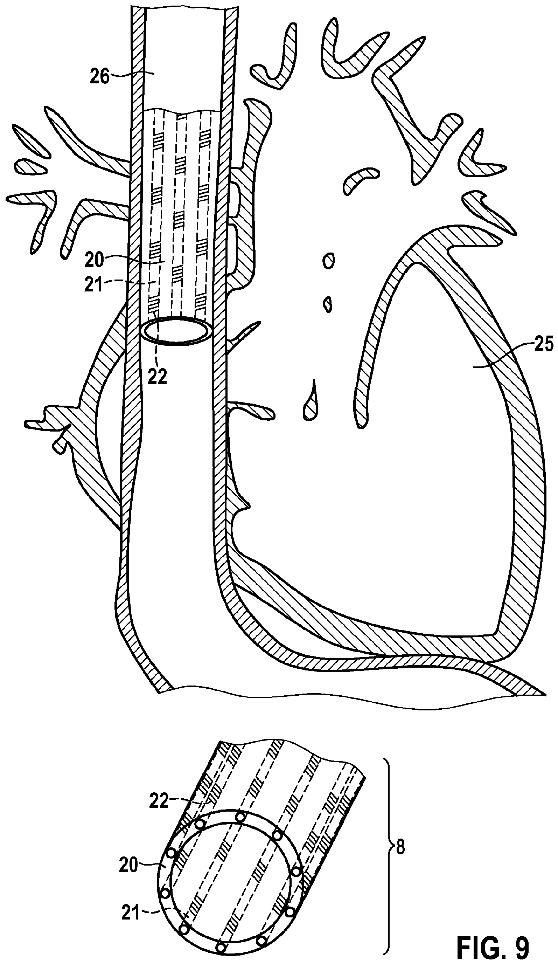

Fig. 9 zeigt im oberen Teil eine Ösophagussonde mit mehreren Lichtwellenleitern 21, die eine einem Mantel des Körpers 20 angeordnet sind und einen Innenraum umgeben. Hierdurch wird eine röhrenförmige Ausführungsform der Ösophagussonde bereitgestellt. Im unteren Teil von Fig. 9 ist eine perspektivische Darstellung der Ösophagussonde 8 gezeigt.

Die hier offenbarten Ausführungsformen der Ösophagussonde können die folgenden Vorteile aufweisen:
- Es sind keine metallischen Bauteile in der Ösophagussonde erforderlich und dadurch erfolgt keine ohmsche Erwärmung.
- Sensoren (z. B. FBGs) im Abstand weniger Millimeter können hochdynamisch ausgelesen werden.
- Anwendungsfehler werden minimiert durch die Verwendung eines gleichen Potentials.
- Wenn mehrere Interferenzfilter eingesetzt werden, kann die Temperatur an verschiedenen Orten bestimmt werden, und es kann eine Temperaturverteilung angegeben werden.

Die in der Beschreibung, den Ansprüchen und den Figuren offenbarten Merkmale können für die Verwirklichung von Ausführungsformen sowohl einzeln als auch in beliebiger Kombination miteinander relevant sein.

### Bezugszeichenliste:

- 1: Lichtwellenleiter (FBG-Faser)
- 1a: Mantel
- 1b: Kern
- 1c: Sensorabschnitt
- 5: Lichtquelle
- 6: Detektor
- 7: Auswerteeinheit
- 8: Ösophagussonde
- 9: Anzeigeeinrichtung
- 10: Patient
- 20: Körper
- 21: Lichtwellenleiter
- 21a: Kern
- 21b: Mantel
- 22: Faser-Bragg-Gitter
- 23: distales Ende des Körpers
- 25: Herz
- 26: Ösophagus (Speiseröhre)
- 27: Schleife
- 28: Korbstruktur
- 29: Ballon
- n1-n3: Brechungsindices
- A: Gitterabstand

## Patentansprüche

1. Ösophagussonde zum Bestimmen der Temperatur und/oder Temperaturverteilung im Ösophagus (26), mit
- einem Körper (20),
- einem zumindest abschnittsweise in dem Körper (20) angeordneten Lichtwellenleiter (21), und
- einem in dem Lichtwellenleiter (21) gebildeten Interferenzfilter (22).

2. Ösophagussonde nach Anspruch 1, wobei der Interferenzfilter (22) ein Faser-Bragg-Gitter ist.

3. Ösophagussonde nach Anspruch 1 oder 2, wobei in dem Lichtwellenleiter (21) mehrere voneinander beabstandete Interferenzfilter (22) gebildet sind.

4. Ösophagussonde nach einem der vorangehenden Ansprüche, wobei in dem Körper (20) mehrere Lichtwellenleiter (21) angeordnet sind, und wobei in jedem der mehreren Lichtwellenleiter (21) wenigstens ein Interferenzfilter (22) gebildet ist.

5. Ösophagussonde nach Anspruch 3 oder 4, wobei die mehreren Interferenzfilter (22) unterschiedliche Filterbandbreiten aufweisen.

6. Ösophagussonde nach einem der vorangehenden Ansprüche, wobei der Lichtwellenleiter (21) zumindest abschnittsweise von einem Mantel umgeben ist.

7. Ösophagussonde nach einem der vorangehenden Ansprüche, wobei der Interferenzfilter (22) von einem Versteifungsmittel umgeben ist.

8. Ösophagussonde nach einem der vorangehenden Ansprüche, ferner ein Markierungselement aufweisend, das eine Position des Interferenzfilters (22) angibt.

9. Ösophagussonde nach einem der vorangehenden Ansprüche, wobei der Körper (20) mit dem Lichtwellenleiter (21) eine Krümmung aufweist.

10. Ösophagussonde nach einem Ansprüche 1 bis 8, wobei der Körper (20) mit dem Lichtwellenleiter (21) spiralförmig ist.

11. Ösophagussonde nach einem Ansprüche 1 bis 8, wobei der Lichtwellenleiter (21) an einem distalen Ende (23) des Körpers (20) eine Schleife (27) bildet.

12. Ösophagussonde nach einem Ansprüche 1 bis 8, wobei in dem Körper (20) mehrere Lichtwellenleiter (21) angeordnet sind, wobei die mehreren Lichtwellenleiter (21) an einem distalen Ende (23) des Körpers (20) eine Korbstruktur (28) bilden und wobei im Bereich der Korbstruktur (28) in jedem der mehreren Lichtwellenleiter (21) wenigstens ein Interferenzfilter (22) gebildet ist.

13. Ösophagussonde nach einem Ansprüche 1 bis 8, wobei in dem Körper (20) mehrere Lichtwellenleiter (21) angeordnet sind, wobei die mehreren Lichtwellenleiter (21) an einem distalen Ende (23) des Körpers (20) auf einem Ballon (29) angeordnet sind und wobei im Bereich des Ballons (29) in jedem der mehreren Lichtwellenleiter (21) wenigstens ein Interferenzfilter (22) gebildet ist.

14. Ösophagussonde nach einem Ansprüche 1 bis 8, wobei in dem Körper (20) mehrere Lichtwellenleiter (21) derart angeordnet sind, dass die mehreren Lichtwellenleiter (21) einen Innenraum umgeben, und wobei in jedem der mehreren Lichtwellenleiter (21) wenigstens ein Interferenzfilter (22) gebildet ist.

15. System mit
- einer Ösophagussonde (8) nach einem der vorangehenden Ansprüche,
- einer Lichtquelle (5), die mit einem proximalen Ende des Lichtwellenleiters (21) gekoppelt ist, und die eingerichtet ist, Licht in den Lichtwellenleiter (21) einzubringen,
- einer Auswerteeinheit (7), die mit dem Lichtwellenleiter (21) gekoppelt ist und die eingerichtet ist, auf Grundlage des von dem Lichtwellenleiter (21) abgegebenen Lichts eine Temperatur zu bestimmen.
